(19) Europäisches Patentamt
European Patent Office
Office européen des brevets

(11) Veröffentlichungsnummer: **0 063 269**
**B1**

# (12) EUROPÄISCHE PATENTSCHRIFT

(45) Veröffentlichungstag der Patentschrift: **11.09.85**

(51) Int. Cl.⁴: **C 07 C 143/58,** C 07 C 143/60, C 07 C 143/63, C 07 C 143/66, C 07 C 139/00

(21) Anmeldenummer: **82102727.3**

(22) Anmeldetag: **31.03.82**

## (54) Verfahren zur Herstellung von Amino-arylsulfonsäuren.

(30) Priorität: **11.04.81 DE 3114830**

(43) Veröffentlichungstag der Anmeldung: **27.10.82 Patentblatt 82/43**

(45) Bekanntmachung des Hinweises auf die Patenterteilung: **11.09.85 Patentblatt 85/37**

(84) Benannte Vertragsstaaten: **CH DE FR GB LI**

(56) Entgegenhaltungen:
**DE - A - 2 312 728**
**DE - C - 549 136**

**Chemical Abstracts, Band 85, Nr. 2, 12. Juli 1976, Columbus, Ohio, USA, T. HAYASHI et al. "Catalytic actions of mercury (II) sulfate and palladium (II) sulfate for the sulfonation of anthraquinone by the sulfur trioxidesulfolane system", Seite 95, abstract nr. 7242u**
**Chemical Abstracts, Band 86, Nr. 26, 27. Juni 1977, Columbus, Ohio, USA, J. MARTINMAA "Sulfolane", Seite 375, Abstract Nr. 195798x**

(73) Patentinhaber: **BAYER AG, Konzernverwaltung RP Patentabteilung, D-5090 Leverkusen 1 Bayerwerk (DE)**

(72) Erfinder: **Emde, Herbert, Dr., Andreas-Gryphiusstrasse 7, D-5000 Köln 80 (DE)**
Erfinder: **Blank, Heinz Ulrich, Dr., Am Geusfelde 35, D-5068 Odenthal (DE)**
Erfinder: **Schnegg, Peter, Dr., Heidbergstrasse 44, D-5068 Odenthal (DE)**

EP 0 063 269 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Amino-arylsulfonsäuren aus einem Arylamin und einem Sulfonierungsagens in Gegenwart von Tetramethylensulfon (Sulfolan).

Es ist bereits bekannt, daß man aromatische Aminosulfonsäuren aus einem Arylamin und Schwefelsäure bei hohen Temperaturen erhält (Helv. Chim. Acta. 15, 1372 [1932]). Dieser sogenannte Backprozeß, bei dem in einer ersten Stufe das zugehörige Arylammoniumsulfat gebildet wird, ist durch Anbackungen und lokale Überhitzungen belastet, die zu teilweisen Verkohlungen, unerwünschten Nebenreaktionen und damit Einbußen der Produktqualität, insbesondere der Produktfarbe, und erhöhten Aufwendungen bei der Aufarbeitung und Reinigung führen. Es ist daher versucht worden, diesen Backprozeß in Gegenwart eines Lösungs- bzw. Verdünnungsmittels durchzuführen, um die gezeigten Mängel aufzuheben oder wenigstens einzuschränken. Als solche Lösungsmittel wurden beispielsweise o-Dichlorbenzol (Ind. Eng. Chem. 42, 1746 [1950]) und Diphenyl-sulfon (Trav. Soc. Sci. Lettres Wroclaw Ser. B. 61, 5 [1953]), zitiert nach C.A. 48, 7568 i [1954]) vorgeschlagen. Die Verwendung von o-Dichlorbenzol bringt jedoch lange Reaktionszeiten von etwa 15 bis 20 Stunden und damit verbundene hohe Energiekosten mit sich. Die Verwendung von Diphenylsulfon beim Backprozeß liefert zum Teil gute Ausbeuten, erfordert jedoch teilweise ebenfalls lange Reaktionszeiten und ist vor allem durch eine aufwendige Abtrennung des Diphenylsulfons gekennzeichnet. Wegen des hohen Schmelzpunkts des Diphenylsulfons von über 100° C ist diese Aufarbeitung nicht durch einfaches Abfiltrieren, sondern nur durch Extraktion mit Benzo möglich und zusätzlich durch die Notwendigkeit der Umkristallisation des Diphenylsulfons vor dem Wiedereinsatz belastet. Gemeinsamer Nachteil der hier beispielhaft erwähnten Lösungs- bzw. Verdünnungsmittel ist weiterhin der Nachteil, daß auch in deren Gegenwart Anbackungen mit nachfolgenden Produktverfärbungen nicht vollständig vermieden werden können, so daß Reinigungsoperationen für das erhaltene Produkt nach wie vor erforderlich sind.

Es wurde nun ein Verfahren zur Herstellung von Aminoarylsulfonsäuren mit freier oder neutralisierter Sulfonsäuregruppe gefunden, das dadurch gekennzeichnet ist, daß in einem Reaktionsmedium, das Tetramethylensulfon und gegebenenfalls weitere inerte Lösungsmittel enthält, ein Arylamin der Formel

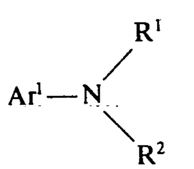

(I)

in der

$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und

$Ar^1$ das gegebenenfalls substituierte Benzol-, Naphthalin-, Anthracen-, Naphthochinon- oder Anthrachinongerüst oder das Gerüst eines aromatischen Heterocyclus darstellt,

mit einem Sulfonierungsagens bei einer Temperatur von 120 bis 280° C ohne Einsatz eines Katalysators umgesetzt wird.

Gegebenenfalls wird das Reaktionsmedium, das nach beendeter Reaktion durch Abtrennung von der gebildeten Amino-arylsulfonsäure in freier oder salzartiger Form zurückgewonnen wird, ohne weitere Reinigung mindestens teilweise in die Reaktion zurückgeführt.

Erfindungsgemäß enthält das Reaktionsmedium 10 bis 2000 ml, bevorzugt 15 bis 1000 ml, besonders bevorzugt 20 bis 500 ml Tetramethylensulfon (Sulfolan) pro Mol Arylamin im Reaktionsmedium.

Erfindungsgemäß kann das Reaktionsmedium neben dem Tetramethylensulfon auch weitere inerte Lösungsmittel, beispielsweise in einer Menge von 1 bis 99 Vol.-%, bevorzugt 10 bis 95 Vol.-%, besonders bevorzugt 50 bis 90 Vol.-%, des gesamten Reaktionsmediums enthalten, wobei die Vorteile der Arbeitsweise in Gegenwart des Tetramethylensulfons voll erhalten bleiben. Geeignete inerte Lösungsmittel sind beispielsweise unsubstituierte bzw. Alkyl-substituierte und/oder Halogen-substituierte Aromaten, wie Toluol, Xylol, Ethylbenzol, Tetralin, Chlorbenzol, Dichlorbenzol, Trichlorbenzol, Tetrachlorbenzol, Chlortoluol, Dichlortoluol, Trichlortoluol, Brombenzol, Dibrombenzol, Bromtoluol oder Dibromtoluol, höhersiedende Aliphaten oder deren Gemische, wie Petroleumfraktionen oder Kerosin, Dekalin, Isododekan, sowie aliphatische Sulfone, wie Dimethylsulfon oder Diethylsulfon. Diese Lösungsmittel können sowohl einzeln als auch im Gemisch neben dem Tetramethylensulfon im Reaktionsmedium eingesetzt werden. Der Einsatz solcher inerten Lösungsmittel kann beispielsweise dazu dienen, durch den Ersatz des teuren Tetramethylensulfons durch preisgünstigere Lösungsmittel das gesamte Verfahren zu verbilligen. Der Zusatz solcher inerten Lösungsmittel kann beispielsweise weiterhin dazu dienen, die bei der Vielzahl der einzusetzenden Substrate sich ergebenden einzelnen Trennprobleme zu optimieren. Solche Anpassungen an die Eigenschaften der einzelnen Substrate sind jeweils durch einfache Vorversuche möglich. Wegen der Einfachheit im Umgang mit nur einem Lösungs- bzw. Verdünnungsmittel ist es jedoch bevorzugt, Tetramethylensulfon ohne weitere inerte Lösungsmittel als Reaktionsmedium einzusetzen. Unabhängig von der Mitverwendung oder der Nichtverwendung weiterer inerter Lösungsmittel braucht das Sulfolan im erfindungsgemäßen Verfahren nicht in wasserfreier Form eingesetzt zu werden.

Im erfindungsgemäßen Verfahren wird ein Arylamin der allgemeinen Formel

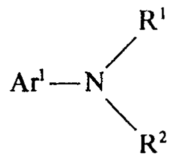

(I)

eingesetzt, in der

$R^1$ und $R^2$ unabhängig voneinander Wasserstoff, Alkyl, Aralkyl oder Aryl bedeuten oder beide gemeinsam mit dem N-Atom, das sie substituieren, einen gegebenenfalls ein oder mehrere weitere Heteroatome enthaltenden Stickstoffheterocyclus bilden, und

$Ar^1$ das gegebenenfalls substituierte Benzol-, Naphthalin-, Anthracen-, Naphthochinon- oder Anthrachinongerüst oder das Gerüst eines aromatischen Heterocyclus darstellt.

Als Alkyl sei beispielsweise ein solches mit 1 bis 8, bevorzugt 1 bis 4, bevorzugt 1 bis 2 C-Atomen genannt, wie Methyl, Ethyl, Propyl, Isopropyl, Butyl, Isobutyl, Hexyl oder Octyl. Als Aralkyl sei beispielsweise Benzyl, 1-Phenyl-ethyl, 2-Phenyl-ethyl, Naphthylmethyl, Naphthylethyl, Anthrylmethyl oder Anthrylethyl, bevorzug Benzyl, genannt.

Als Aryl sei beispielsweise Phenyl, substituiertes Phenyl, Naphthyl oder Diphenyl, bevorzugt Phenyl, genannt.

Für den Fall, daß $R^1$ und $R^2$ gemeinsam mit dem N-Atom, das sie substituieren, einen Stickstoffheterocyclus bilden, sei ein solcher mit beispielsweise 4 bis 8, bevorzugt 5 oder 6 Ringgliedern genannt, wie Pyrrolin, Pyrrolidin oder Piperidin.

Für den Fall, daß $Ar^1$ ein substituiertes Benzol-, Naphthalin-, Anthracen-, Naphthochinon- oder Anthrachinongerüst darstellt, sei neben der Aminogruppe $-NR^1R^2$ beispielsweise eine Zahl bis zu drei weiteren Substituenten, bevorzugt bis zu zwei Substituenten genannt, wobei die Substituenten so angeordnet sind, daß mindestens eine ortho- oder p-Stellung unsubstituiert ist. Als Substituenten seien beispielsweise genannt: Alkyl im Rahmen des obengenannten Bedeutungsumfanges, Trifluormethyl, Perfluorethyl, weiterhin Phenyl, Alkoxy mit 1 bis 4, bevorzugt 1 bis 2 C-Atomen, beispielsweise Methoxy, Ethoxy, Propoxy, Isopropyloxy, Butoxy oder Isobutyloxy, Alkylthio, beispielsweise die Thioanaloga der genannten Alkoxygruppen, Halogen, wie Fluor, Chlor oder Brom, weiterhin Hydroxy, Nitro, gegebenenfalls substituiertes Amino, $SO_3H$ oder Carboxyl sowie Alkylsulfonyl oder Arylsulfonyl. Als Substituenten seien bevorzugt Methyl, Ethyl, Halogenmethyl, Phenyl, Methoxy, Ethoxy, Halogen, Hydroxyl, Nitro, gegebenenfalls substituiertes Amino, $SO_3H$ oder Carboxyl sowie Alkylsulfonyl oder Arylsulfonyl, wie Methyl-, Ethyl- oder Phenylsulfonyl genannt. Ganz besonders bevorzugt als Substituenten seien Methyl, Chlor, Brom, Fluor, Methoxy oder Ethoxy genannt.

Bevorzugte, erfindungsgemäß einsetzbare Arylamine sind solche der Formel

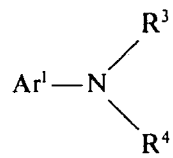

(II)

in der

$Ar^1$ die genannte Bedeutung hat und
$R^3$ und $R^4$ unabhängig voneinander für Wasserstoff oder Alkyl stehen.

Besonders bevorzugte Arylamine für das erfindungsgemäße Verfahren sind solche der Formel

$$Ar^1-NH_2 \quad (III)$$

in der
$Ar^1$ die genannte Bedeutung hat.

Weitere bevorzugte Arylamine für das erfindungsgemäße Verfahren sind solche der Formel

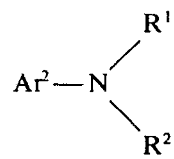

(IV)

in der

$R^1$ und $R^2$ die obengenannte Bedeutung haben und
$Ar^2$ das gegebenenfalls substituierte Benzol- oder Naphthalingerüst darstellt.

Weitere besonders bevorzugte Arylamine für das erfindungsgemäße Verfahren sind solche der Formel

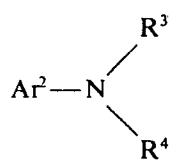

(V)

in der
$R^3$, $R^4$ und $Ar^2$ die genannte Bedeutung besitzen.

In ganz besonders bevorzugter Weise werden Arylamine der Formel

$$Ar^2-NH_2 \quad (VI)$$

eingesetzt, in der
$Ar^2$ die genannte Bedeutung besitzt.

Beispiele für im erfindungsgemäßen Verfahren einsetzbare Arylamine sind

Anilin, o-Toluidin,
m-Toluidin, p-Toluidin,
2,4-Dimethyl-anilin,
2,3-Dimethyl-anilin,
2,6-Dimethyl-anilin,
2,5-Dimethyl-anilin,
N-Methyl-anilin,
N-Ethylanilin,
N,N-Dimethyl-anilin,
Diphenylamin,
p-Chloranilin,
2,4-Dichlor-anilin,
o-Chloranilin,
2,3-Dichloranilin,
3,5-Dichloranilin,
2,5-Dichloranilin,
2,6-Dichloranilin,
m-Chloranilin,
2-Amino-6-chlor-toluol,
2-Amino-5-chlor-toluol,
2-Amino-4-chlor-toluol,
2-Methoxy-5-methyl-anilin,
p-Methoxy-anilin,
o-Nitroanilin,
m-Nitroanilin,
p-Nitroanilin,
α-Naphthylamin,
p-Phenylendiamin,
m-Phenylendiamin,
Amino-diphenyl,
p-Nitrodiphenylamin,
2-Methoxy-4-nitro-anilin,
1-Amino-2-ethoxy-naphthalin,
1-Amino-2-hydroxy-naphthalin,
1-Amino-8-hydroxy-naphthalin,
1-Amino-5-hydroxy-naphthalin,
1,8-Diamino-naphthalin,
1,5-Diamino-naphthalin,
2-Amino-3-hydroxy-naphthalin,
2-Amino-pyridin,
3-Chlor-4-methoxy-anilin,
2-Aminobenzoesäure,
p-Ethoxy-anilin,
3,4-Dichloranilin,
o-Fluoranilin,
m-Fluoranilin,
p-Fluoranilin,
2-Amino-3-chlortoluol,
3-Amino-2-chlortoluol,
5-Amino-2-chlortoluol,
3-Amino-5-chlortoluol,
3-Amino-4-chlortoluol,
4-Amino-3-chlortoluol,
4-Amino-2-chlortoluol,
5-Methoxy-2-methylanilin,
2,3-, 2,4-, 2,5-, 2,6-, 3,4-,
3,5-Dimethoxy- und
Diethoxyanilin,
o-Methoxy-anilin,
m-Methoxy-anilin,
N-Acetyl-p-Phenylendiamin,
2-Chlor-4-methoxy-anilin,
2-Chlor-3-methoxy-anilin,
4-Chlor-3-methoxy-anilin,
5-Chlor-3-methoxy-anilin,
2-Chlor-5-methoxy-anilin,
3-Chlor-2-methoxy-anilin,
4-Chlor-2-methoxy-anilin,
5-Chlor-2-methoxy-anilin,
2-Chlor-6-methoxy-anilin
sowie die analogen
Chlor-ethoxy-aniline,
3-Amino-6-chlor-benzoesäure,
o-Trifluormethyl-anilin,
m-Trifluormethylanilin,
p-Trifluormethyl-anilin,
Amino-anthrachinone wie z. B.
1-Aminoanthrachinon oder
1,5-Diaminoianthrachinon,
Benzidin und
Dehydrothiotoluidin.

Als Sulfonierungsagentien für das erfindungsgemäße Verfahren seien beispielsweise genannt: Schwefelsäure, Halogensulfonsäuren, wie Fluorsulfonsäure und Chlorsulfonsäure, Schwefeltrioxid, Amidosulfonsäure oder saure Salze der Schwefelsäure bzw. ein Gemisch aus einem Sulfat und Schwefelsäure.

Erfindungsgemäß werden beispielsweise 0,5 bis 2,0 Mol Sulfonierungsagens pro Mol Arylamin eingesetzt, bevorzugt 0,95 bis 1,05, besonders bevorzugt 0,98 bis 1,02, ganz besonders bevorzugt 1 Mol Sulfonierungsagens pro Mol Arylamin.

Die genannten Sulfonierungsagentien können einzeln eingesetzt werden. Ein Teil der genannten Agentien kann jedoch auch als Gemisch eingesetzt werden, so beispielsweise verschiedene Halogensulfonsäuren untereinander, Schwefelsäure mit einer oder mehreren Halogensulfonsäuren und Schwefelsäure mit Schwefeltrioxid als Oleum mit 1 bis 65 Gew.-% $SO_3$. Weiterhin sind Gemische aus Schwefelsäure und Schwefeltrioxid einsetzbar, in denen der Anteil an Schwefelsäure 0—35% betragen kann. In vielen Fällen wird es jedoch wegen der Übersichtlichkeit der Reaktionsführung vorteilhaft sein, nur eines der genannten Sulfonierungsagentien einzusetzen. Für den Fall, daß Schwefelsäure als Sulfonierungsagens eingesetzt wird, kann diese einen Wassergehalt von 0 bis 50 Gew.-%, bevorzugt 0 bis 30 Gew.-% und besonders bevorzugt 0 bis 10 Gew.-%, haben. Dieses Verdünnungswasser der Schwefelsäure kann bei der Durchführung der erfindungsgemäßen Reaktion zusammen mit dem aus den Substraten entstehenden Reaktionswasser aus dem Reaktionsansatz herausdestilliert werden. Bei dieser Verfahrensvariante kann beispielsweise eines der obengenannten Lösungsmittel als Schleppmittel bei der Entfernung des Wassers aus dem Reaktionsgemisch dienen. Das Herausdestillieren des Verdünnungswassers und/oder Reaktionswassers ist jedoch grundsätzlich auch ohne ein zusätzliches Wasserschleppmittel möglich. Für den Fall, daß Halogensulfonsäuren als Sulfonierungsagens eingesetzt werden, wird zur Vervollständigung der Reaktion der gebildete Halogenwasserstoff aus dem Reaktionsansatz abgeleitet. Für den Fall, daß $SO_3$ als Sulfonierungsagens eingesetzt wird, kann dieses in gasförmiger oder flüssiger Form eingesetzt werden, wobei beim Einsatz in gasförmiger Form das $SO_3$ mit einem Inertgas, wie Stickstoff, $CO_2$ oder Argon, vermischt werden kann und wobei beim Einsatz in flüssiger Form zusätzlich ein Lösungsmittel, beispielsweise Schwefelsäure in Form des bereits genannten Oleums oder auch eines der weiter oben genannten Lösungsmittel verwendet werden können. Bei der Anwendung von Schwefeltrioxid braucht weder Wasser noch Halogenwasserstoff als Kondensationsprodukt abgespalten zu werden. Ferner genügt hierbei in den meisten Fällen eine sehr kurze Reaktionszeit. Für den Fall, daß Schwefeltrioxid als Sulfonierungsagens eingesetzt wird und das eingesetzte Lösungsmittel wasserhaltig ist, kann ein Überschuß an $SO_3$ eingesetzt werden oder aber man muß für einen Teil der Sulfonierungsreaktion Bedingungen wählen, wie sie beim Einsatz von Schwefelsäure als Sulfonierungsagens notwendig sind.

Beim Einsatz von Amidosulfonsäure können in einfacher Weise anstelle der freien Sulfonsäure die Ammoniumsalze der Sulfonsäure gewonnen werden. Es wurde beobachtet, daß unter den erfindungsgemäßen Bedingungen 2 Mol Amidosulfonsäure eingesetzt werden müssen, um 1 Mol hiervon als wirkungsvolles Sulfonierungsagens zur Verfügung zu haben. Die obengenannten Mengen Sulfonierungsagens pro Mol Arylamin müssen daher im Falle der Amidosulfonsäure verdoppelt werden.

Beim Einsatz eines sauren Salzes der Schwefelsäure bzw. eines Sulfates im Gemisch mit Schwefelsäure können ebenfalls in eleganter und direkter Weise die Aminoarylsulfonsäuren hergestellt werden. Das Gemisch besteht hierbei aus etwa äquimolaren Mengen des Sulfates und der Schwefelsäure. Als erfindungsgemäß einsetzbare Hydrogensulfate bzw. Sulfate seien hierbei beispielsweise die löslichen Salze der Alkalimetalle und Erdalkalimetalle, sowie des Ammoniumions genannt, wie die des Lithiums, Natriums, Kaliums, Rubidiums, Cäsiums, Ammoniums, Magnesiums und Calciums, bevorzugt die Salze des Ammoniums, Natriums oder Kaliums.

In bevorzugter Weise wird als Sulfonierungsagens mindestens eines aus der Gruppe der Schwefelsäure des $SO_3$ und der Chlorsulfonsäure eingesetzt, wobei beim Einsatz von Schwefelsäure allein diese einen Wassergehalt, wie oben offenbart, bevorzugt 0 bis 30 Gew.-%, haben kann.

Die Reaktionspartner können in verschiedener Reihenfolge im Reaktionsgefäß zusammengegeben werden. So kann entweder das Arylamin oder das Sulfonierungsagens in Tetramethylensulfon vorgelegt werden und die jeweils zweite Komponente in reiner Form oder mit Tetramethylensulfon oder einem anderen der genannten Lösungsmittel verdünnt zugegeben werden. Ebenso ist eine gleichzeitige Dosierung der beiden Komponenten einzeln oder gemeinsam in Form einer Lösung oder einer Schmelze möglich, wobei das Tetramethylensulfon entweder im Reaktor vorgelegt wird oder als Verdünnungs- bzw. Lösungsmittel mit dem Gemisch der Reaktionspartner oder aufgeteilt auf die Einzelströme der Reaktionspartner zugegeben wird.

Die auf eine der beschriebenen Weisen hergestellte Suspension oder Lösung aus den Ausgangsprodukten und dem Tetramethylensulfon oder weiteren Lösungsmitteln wird sodann auf beispielsweise 120 bis 280°C, bevorzugt auf 150 bis 260°C, besonders bevorzugt auf 160 bis 240°C, erhitzt, wobei gleichzeitig das gegebenenfalls abgespaltene Wasser oder der gegebenenfalls abgespaltene Halogenwasserstoff aus dem Reaktionsmedium entfernt wird.

In einer weiteren Variante können auch das Tetramethylensulfon sowie gegebenenfalls mitverwendetes weiteres inertes Lösungsmittel auf eine Temperatur im genannten Bereich erhitzt werden und die Ausgangsstoffe in Form einer Schmelze, Suspension oder Lösung in das heiße vorgelegte Reaktionsmedium eingetragen werden.

Das Verfahren kann kointinuierlich oder diskontinuerlich gestaltet werden.

Das Verfahren kann grundsätzlich in Vakuum, bei Normal- oder bei Überdruck durchgeführt werden. Gelegentlich kann ein Überdruck vorteilhaft sein, beispielsweise um in Gegenwart eines niedrigsiedenden Lösungsmittels eine höhere Reaktionstemperatur zu erreichen. Gelegentlich kann jedoch auch das Anlegen von Vakuum zweckmäßig sein, beispielsweise bei hochsiedenden Lösungsmitteln, wenn unter Rückflußbedingungen gearbeitet werden soll oder bei der Sulfonierung von temperaturempfindlichen Arylaminen.

Zur Aufarbeitung des Reaktionsansatzes und zur Abtrennung der gebildeten Amino-arylsulfonsäure sind verschiedene Varianten möglich, wobei die Aminoarylsulfonsäure in freier oder in salzartiger Form gewonnen wird. So kann in vielen Fällen die ausgefallene Amino-arylsulfonsäure durch einfache Filtration vom Reaktionsmedium abgetrennt werden. Für den Fall, daß Tetramethylensulfon ohne weiteres inertes Lösungsmittel als Reaktionsmedium angewandt wird, kann ein vollständiges Ausfällen der gebildeten Amino-arylsulfonsäure auch durch Zusatz von Wasser erfolgen. In diesem Falle wird beispielsweise nach dem Abfiltrieren das Tetramethylensulfon aus dem Filterkuchen mit Wasser verdrängt. Für den Fall, daß nicht bereits während der Umsetzung das Salz der Amino-arylsulfonsäure nach einer der beschriebenen Varianten erhalten wurde, kann dies bei der Aufarbeitung durch Zusatz von wäßrigem Alkali zum Reaktionsansatz geschehen, wobei ein Niederschlag des korrespondierenden Salzes der Amino-arylsulfonsäure erhalten wird. Als wäßriges Alkali für diese Variante sei beispielsweise eine Lösung oder Suspension von Ammoniak, Lithiumhydroxid, Natriumhydroxid, Kaliumhydroxid, Magnesiumoxid oder -hydroxid oder Calciumhydroxid genannt. In einer weiteren Variante läßt sich durch Einbringen von Ammoniak oder einem aliphatischen Amin in den Reaktionsansatz auch bei wasserfreier Aufarbeitung eine Ausfällung der Ammoniumsalze der Amino-arylsulfonsäuren erreichen.

Das bei einer der genannten Aufarbeitungsvarianten als Filtrat gewonnene Tetramethylensulfon, gegebenenfalls im Gemisch mit einem weiteren inerten Lösungsmittel kann ohne weitere Reinigung wieder in das erfindungsgemäße Verfahren zurückgeführt werden. Für den Fall, daß als Sulfonierungsagens $SO_3$ oder eine Halogensulfonsäure oder ein Gemisch, das diese enthält, verwendet wird, ist es jedoch zweckmäßig, etwa im wiederzuverwendenden Tetramethylensulfon vorhandenes Wasser vor der Wiederverwendung auszudestillieren, um eine Zersetzung der genannten Sulfonierungsagentien weitgehend zu verhindern. Für den Fall, daß beispielsweise Schwefelsäure oder eine wasserhaltige Schwefelsäure als Sulfonierungsagens eingesetzt wird, stört jedoch ein geringer Wassergehalt des zurückgeführten Tetramethylensulfons nicht und kann beim nächsten Reaktionsansatz gemeinsam mit dem Reaktionswasser ausdestilliert werden. Sollte das wiedergewonnene Tetramethylensulfon jedoch einen größeren Wassergehalt haben, kann es zweckmäßig sein, dieses Wasser oder wenigstens einen Teil hiervon vor der Wiederverwendung herauszudestillieren. Filtrate, die beim Verdrängen des Tetramethylensulfons aus dem Filterkuchen mit Hilfe von Wasser erhalten werden, werden zweckmäßigerweise getrennt aufgefangen und soweit wie möglich bei den oben beschriebenen Aufarbeitungsvarianten anstelle des Zusatzes von reinem Wasser zum Reaktionsgemisch verwendet.

Das erfindungsgemäße Verfahren erlaubt es, die Nachteile der bisher bekanntgewordenen Varianten des sogenannten Backverfahrens zu umgehen, nämlich die zum Teil sehr langen Reaktionszeiten, die Anbackungen an den Reaktorwänden, die damit verbundenen Produktschädigungen und zusätzlich erforderlichen Reinigungsstufen und die damit verbundenen, oft nur sehr geringen Ausbeuten, insbesondere bei thermisch empfindlichen Aminen, sowie die Isomerenbildung und die Disulfonierung.

Tetramethylensulfon bzw. Tetramethylensulfon-haltige Lösungs- bzw. Verdünnungsmittel liefern im Gegensatz hierzu erfindungsgemäß äußerst reine Produkte in sehr kurzen Reaktionszeiten. Bezogen auf das Tetramethylensulfon bzw. seinen Gehalt im Reaktionsmedium, kann in sehr hohen Amin/Sulfonierungsagens-Konzentrationen gearbeitet werden, ohne daß Rühr- oder Anbackprobleme auftreten. Dadurch werden ausgezeichnete Raum-Zeit- und Material-Ausbeuten erhalten. Die Unempfindlichkeit von Tetramethylensulfon gegen Säuren und Basen und seine geringe Toxizität sind weiterhin vorteilhaft in bezug auf die Materialauswahl des Reaktors und in bezug auf arbeitshygienische Bedingungen (J. Martinmaa, The Chemistry of Non-Aqueous Solvents; Academic Press, New York 1976, Vol. IX, S. 247 ff.). Für den Fall, daß in Tetramethylensulfon als Reaktionsmedium ohne weitere inerte Lösungsmittel gearbeitet wird, ist als weiterer Vorteil zu nennen, daß beim Herausdestillieren von Wasser das Tetramethylensulfon nicht im Destillat auftaucht, was bei den bisher nach dem Stande der Technik verwendeten Lösungsmitteln der Fall ist.

Für die Reinigung und Rückführung dieser mitherausdestillierten Lösungsmittel mußte bisher ein beträchtlicher Energieaufwand getrieben werden, der beim erfindungsgemäßen Verfahren fortfällt.

Die im erfindungsgemäßen Verfahren erhältlichen Amino-arylsulfonsäuren sind wertvolle Zwischenprodukte für die Herstellung von Pharmazeutika, Schaumstoffen, optischen Aufhellern, Netzmitteln, synthetischen Beizmitteln, Gerbstoffen, Reservierungsmitteln, Insektiziden, Apreturmitteln, Weichmachern und polymeren Verdickungsmitteln (Ullmanns Encyclopädie der technischen Chemie, 3. Auflage, Band 16, Seite 561, Verlag Urban und Schwarzenberg, München/Berlin 1965).

## Beispiel 1

232,8 g (2,50 Mol) Anilin werden in 500 ml 85°C heißem Sulfolan vorgelegt und 245,3 g (2,50 Mol) 100%ige Schwefelsäure ohne Kühlung zugetropft. Die Temperatur läßt man dabei bis auf 160°C ansteigen. Die gut rührbare Aniliniumsulfatsuspension schmilzt bei Aufheizen bei etwa 165°C. Das Reaktionswasser wird im Laufe von 1,5 Stunden bei einer Innentemperatur von 180—192°C abdestilliert. Die p-Sulfanilsäure fällt als weißes, feinkristallines Produkt an. Man saugt ab und trocknet bei 130°C und 200 Torr. Das Gewicht des Niederschlags beträgt 431,3 g, die Ausbeute an p-Sulfanilsäure 98,9%, und der Gehalt liegt bei 99,3 Gew.-%.

## Beispiel 2

232,8 g (2,50 Mol) Anilin werden in 500 ml 85°C heißem Sulfonlan vorgelegt und 255,5 g (2,50 Mol) 96%ige Schwefelsäure ohne Kühlung zugetropft. Die Durchführung der Sulfonierung und die Aufarbeitung erfolgt, wie in Beispiel 1 beschrieben. Die Ausbeute an p-Sulfanilsäure beträgt 94,4%.

## Beispiel 3

232,8 g (2,50 Mol) Anilin werden in 500 ml 85°C heißem Sulfonlan vorgelegt und 350,4 g (2,50 Mol) 70%ige Schwefelsäure ohne Kühlung zugetropft. Die Durchführung der Sulfonierung und die Aufarbeitung erfolgt, wie in Beispiel 1 beschrieben. Die Ausbeute an p-Sulfanilsäure beträgt 95,9%.

Beispiel 4

232,8 g (2,50 Mol) Anilin werden in 500 ml 85°C heißem Sulfolan vorgelegt und 257,5 g (2,625 Mol) 100%ige $H_2SO_4$ ohne Kühlung zugetropft; man heizt auf 178°C auf und destilliert das Reaktionswasser ab, wobei die Temperatur bis auf 195°C ansteigt.

Die Ausbeute an p-Sulfanilsäure beträgt 97,2%, bez. auf Anilin.

Beispiel 5

247,7 g (2,525 Mol) Anilin werden in 500 ml Sulfolan mit 245,3 g (2,50 mol) 100%ige $H_2SO_4$, wie in Beispiel 1 beschrieben, umgesetzt. Es wird eine Ausbeute an p-Sulfanilsäure von 95,9% erzielt.

Beispiel 6

232,8 g (2,50 Mol) Anilin werden in 250 ml 85°C heißem Sulfolan vorgelegt und 245,3 g (2,50 Mol) 100%ige $H_2SO_4$ ohne Kühlung in 18 Minuten zugetropft. Das Reaktionswasser wird in 45 Minuten bei 195°C Reaktionstemperatur abdestilliert. Die dickflüssige Suspension wird bei 100°C mit 300 ml Wasser versetzt und abgesaugt. Von der Mutterlauge wird das Waschwasser abdestilliert und die ausfallende p-Sulfanilsäure auch abgesaugt. Die Niederschläge werden gemeinsam getrocknet. Die Ausbeute an p-Sulfanilsäure beträgt 95,7%.

Beispiel 7

93,1 g (1,0 Mol) Anilin werden in 500 ml Sulfolan bei Raumtemperatur vorgelegt. Bei dieser Temperatur werden 80,1 g gasförmiges Schwefeltrioxid in einer Stunde eingeleitet, und nach beendeter Zugabe wird die Temperatur auf 180°C erhöht. Es wird 2 Stunden bei dieser Temperatur gerührt. Die Aufarbeitung erfolgt wie in Beispiel 1 beschrieben. Die Ausbeute an p-Sulfanilsäure beträgt 96,6%.

Beispiel 8

93,1 g (1,0 Mol) Anilin werden in 500 ml Sulfolan bei Raumtemperatur vorgelegt. Bei dieser Temperatur werden 80,1 g (1,0 Mol) gasförmiges Schwefeltrioxid über die Lösung geleitet. Anschließend wird die Suspension 1 Stunde bei 195°C gerührt, wobei eine dicke Suspension entsteht. Man versetzt mit 200 ml Wasser und saugt den Niederschlag ab. Die Ausbeute an p-Sulfanilsäure beträgt 95,1%.

Beispiel 9

93,1 g (1,0 Mol) Anilin werden bei 150°C in 500 ml Sulfolan vorgelegt. In 18 Minuten werden 116,5 g (0,98 Mol) Chlorsulfonsäure (98%ig) zugetropft, wobei gegen Ende der Reaktion starke HCl-Entwicklung einsetzt. Man rührt 6 Stunden unter Rückfluß nach, wobei die Temperatur von 177°C auf 161°C sinkt. Die ausgefallene p-Sulfanilsäure wird abgesaugt, die Mutterlauge eingeengt und beide Niederschläge getrocknet. Die Ausbeute an p-Sulfanilsäure beträgt 97,2%.

Beispiel 10

186,2 g (2,0 Mol) Anilin werden in 500 ml Sulfolan bei Raumtemperatur vorgelegt, und in 13 Minuten werden 116,5 g (0,98 Mol) Chlorsulfonsäure (98%ig) zugetropft, wobei die Temperatur auf 100°C ansteigt. Die weiße, dicke Suspension wird 2 Stunden am Rückfluß erhitzt, wobei die Temperatur von 172°C auf 180°C ansteigt. Die Aufarbeitung erfolgt wie in Beispiel 9 beschrieben. Die Ausbeute beträgt 95,5%.

Beispiel 11

93,1 g (1,0 Mol) Anilin werden zusammen mit 195,4 g (2,0 Mol) Amidosulfonsäure (99,3%ig) in 500 ml Sulfolan auf 173°C erhitzt. Man rührt noch 2 Stunden nach, wobei eine Temperatur auf 135°C fällt. Der Niederschlag wird bei 120°C abgesaugt und getrocknet. Er besteht zum größten Teil aus dem Ammoniumsalz der p-Sulfanilsäure. Die Ausbeute, gerechnet als p-Sulfanilsäure, beträgt 92,8%.

Beispiel 12

127,6 g (1,0 Mol) o-Chloranilin werden bei Raumtemperatur in 500 ml Sulfolan gelöst, vorgelegt und 98,1 g (1,0 Mol) 100%ige $H_2SO_4$ ohne Kühlung zugetropft. Die hellgelbe, 76°C warme Suspension wird auf 180°C erhitzt und bei dieser Temperatur über eine 20-cm-Silbermantelkolonne das Reaktionswasser abdestilliert. Die Temperatur steigt im Laufe der 1,5stündigen Reaktionszeit auf 194°C an. Mit dem Wasser destillieren 3 ml = 3,6 g (0,03 Mol) O-Chloranilin über, das sich durch Phasentrennung zurückgewinnen läßt. Die Suspension wird auf 160°C abgekühlt, abgesaugt und getrocknet. Die Ausbeute an 4-Amino-3-chlor-benzolsulfonsäure beträgt 93,8%.

Beispiel 13

127,6 g (1,0 Mol) m-Chloranilin werden in 500 ml Sulfolan vorgelegt und in 9 Minuten 98,1 g (1,0 Mol) 100%ige $H_2SO_4$ zugetropft. Die Temperatur steigt auf 81°C an, und es fällt ein hellbeige-farbiger Feststoff aus. Es wird auf 218°C erhitzt, wobei alles in Lösung geht. Im Laufe von 2,5 Stunden wird bei dieser Temperatur das Reaktionswasser abdestilliert. Man kühlt auf Raumtemperatur ab und saugt den Niederschlag ab. Die Ausbeute an 2-Amino-4-chlor-benzolsulfonsäure beträgt 89,7%.

Beispiel 14

127,6 g (1,0 Mol) p-Chloranilin werden bei 85°C in 500 ml Sulfolan vorgelegt und in 10 Minuten 98,1 g (1,0 Mol) 100%ige $H_2SO_4$ zugetropft, wobei die Temperatur auf 123°C ansteigt. Die braune Lösung wird auf 172°C erhitzt und bei einem Unterdruck von 130—150 Torr und einer Temperatur von 172—196°C das Reaktionswasser abdestilliert. Die Suspension wird auf 160°C abgekühlt und abgesaugt. Die Ausbeute an 2-Amino-5-chlor-benzolsulfonsäure beträgt 95,7%.

Beispiel 15

107,2 g (1,0 Mol) o-Toluidin werden in 500 ml Sulfolan gelöst und in 8 Minuten mit 98,1 g (1,0 Mol) 100%iger $H_2SO_4$ versetzt. Während des Zutropfens steigt die Temperatur auf 77°C an. Zu Beginn der Schwefelsäurezugabe entsteht eine Suspension, am Ende der Zugabe liegt eine Lösung vor. In 1,5 Stunden wird bei einer Reaktionstemperatur von 184—200°C das Reaktionswasser abdestilliert. Danach kühlt man die Suspension auf 120°C ab und saugt ab. Die Ausbeute an 4-Amino-3-methyl-benzolsulfonsäure beträgt 93,8%.

Beispiel 16

107,2 g (1,0 Mol) m-Toluidin werden in 500 ml Sulfolan vorgelegt, innerhalb von 10 Minuten tropft man 98,1 g (1,0 Mol) 100%ige $H_2SO_4$ zu, wobei die Temperatur auf 81°C ansteigt und ein hellbeiger Niederschlag ausfällt. Beim Aufheizen auf 191°C Reaktionstemperatur geht der Feststoff in Lösung. Bei einer Temperatur von 191—216°C wird in 2 Stunden das Reaktionswasser abdestilliert. Die entstandene Suspension wird auf 160°C abgekühlt und abgesaugt. Die Ausbeute an 4-Amino-2-methyl-benzolsulfonsäure beträgt 86,6%.

Beispiel 17

107,2 g (1,0 Mol) p-Toluidin werden in 500 ml Sulfolan vorgelegt und 98,1 g (1,0 Mol) 100%ige $H_2SO_4$ in 10 Minuten zugegeben. Die nun 80°C warme Suspension wird auf 178°C gebracht, wobei der Feststoff in Lösung geht. Innerhalb von 3 Stunden wird bei 178—198°C das Reaktionswasser abdestilliert. Von der Suspension werden 200 ml Sulfolan abdestilliert. Anschließend auf Raumtemperatur abgekühlt und abgesaugt. Die Ausbeute an 2-Amino-5-methyl-benzolsulfonsäure beträgt 89,6%.

Beispiel 18

141,5 g (1,0 Mol) 4-Amino-2-chlor-toluol werden in 500 ml Sulfolan vorgelegt und 99,1 g (1,01 Mol) 100%ige $H_2SO_4$ in 15 Minuten zugetropft, wobei es zu einem Temperaturanstieg auf 80°C kommt. Es wird auf 207°C erhitzt und im Laufe von 1,5 Stunden das Reaktionswasser abdestilliert, wobei die Temperatur auf 218°C ansteigt. Die Suspension wird auf Raumtemperatur abgekühlt. Die Ausbeute an 2-Amino-4-chlor-5-methyl-benzolsulfonsäure beträgt 91,0%.

Beispiel 19

141,5 g (0,99 Mol) 99%iges 5-Amino-2-chlortoluol werden in 500 ml Sulfon gelöst und mit 99,1 g (1,01 Mol) 100%ige $H_2SO_4$ in 10 Minuten versetzt, wobei die Temperatur auf 86°C ansteigt. Das Reaktionswasser wird in 1,5 Stunden bei 207—220°C abdestilliert. Die Suspension wird auf Raumtemperatur abgekühlt und abgesaugt. Die Ausbeute an 2-Amino-5-chlor-4-methyl-benzolsulfonsäure beträgt 85,2%.

Beispiel 20

137,1 g (1,0 Mol) 2-Methoxy-5-methyl-anilin werden bei 85°C gelöst in 500 ml Sulfolan vorgelegt und 98,1 g (1,0 Mol) 100%ige $H_2SO_4$ angegeben. Man legt ein Vakuum von 160—100 mbar an, erhitzt auf 152—163°C und destilliert in 1,5 Stunden das Reaktionswasser ab. Die Suspension wird bei 150°C abgesaugt und getrocknet. Die Ausbeute an 4-Amino-5-methoxy-2-methylbenzolsulfonsäure beträgt 98,5%.

Beispiel 21

121,2 g (1,0 Mol) 2,4-Dimethyl-anilin werden in 500 ml Sulfolan vorgelegt und 98,1 g (1,0 Mol) 100%ige $H_2SO_4$ ohne Kühlung zugetropft. Man heizt langsam auf 188°C auf, reduziert hierbei den Druck von 170 auf 118 mbar und destilliert in 2 Stunden das Reaktionswasser ab. Der Ansatz wird zur Trockne eingeengt. Die Ausbeute an 2-Amino-3,5-dimethyl-benzolsulfonsäure beträgt 87,0%.

Beispiel 22

94,5 g (0,66 Mol) $\alpha$-Naphthylamin werden in 500 ml Sulfolan gelöst, und bei maximal 90°C werden 64,7 g (0,66 Mol) 100%ige $H_2SO_4$ zugetropft. Die Lösung wird bei 110 mbar auf 180°C erhitzt und in 2 Stunden das Reaktionswasser abdestilliert. Die Suspension wird in der Hitze abfiltriert und getrocknet. Ausbeute an 4-Aminonaphthalin-1-sulfonsäure beträgt 89%.

Beispiel 23

159,2 g (1,0 Mol) 5-Amino-1-hydroxy-naphthalin werden in 500 ml Sulfolan bei 57°C gelöst und in 10 Minuten mit 98,1 g (1,0 Mol) 100%iger $H_2SO_4$ versetzt. Die Temperatur steigt auf 86°C an. In 2,75 Stunden wird bei 172—190°C das Reaktionswasser abdestilliert, die entstandene Suspension bei 120°C abgesaugt und der Rückstand getrocknet. Die Ausbeute an 4-Amino-8-hydroxy-naphthalin-1-sulfonsäure beträgt 71,2%.

Beispiel 24

232,8 g (2,50 Mol) Anilin werden in einer Mischung aus 50 ml Sulfolan und 450 ml 1,2,4-Trichlorbenzol vorgelegt und mit 245,3 g (2,50 Mol) 100%ige $H_2SO_4$ ohne Kühlung versetzt. Das Reaktionswasser wird bei einer Temperatur von 174—186°C in 4,5 Stunden über eine 13-cm-Kolonne abdestilliert. Man kühlt auf 120°C ab und saugt die Suspension ab. Die Ausbeute an p-Sulfanilsäure beträgt 96,3%.

Beispiel 25

235,1 g (2,525 Mol) Anilin werden in einer Mischung aus 150 ml Sulfolan und 350 ml 1,2-Dichlorbenzol gelöst und in 16 Minuten mit 245 g (2,50 Mol) 100%iger $H_2SO_4$ versetzt. Man heizt auf 168°C und destilliert im Laufe von 2 Stunden das Reaktionswasser mit Lösungsmittel ab, wobei gegen Ende der Destillation die Temperatur auf 180°C gesteigert wird. Die helle Suspension wird bei 150°C abgesaugt und getrocknet. Die Ausbeute an p-Sulfanilsäure beträgt 93,8%.

Beispiel 26

141 g (1,52 Mol) Anilin werden in einer Mischung von 350 ml Isododekan und 150 ml Sulfolan vorgelegt und mit 147 g (1,50 Mol) 100%iger $H_2SO_4$ versetzt. Der Reaktionsansatz wird 1,5 Stunden bei 172—177°C am Wasserabscheider erhitzt. Der Feststoff wird bei Raumtemperatur abgesaugt und getrocknet. Die Ausbeute an p-Sulfanilsäure beträgt 96,5%.

Beispiel 27

94,0 g (1,01 Mol) Anilin werden in einer Mischung von 400 ml Decalin und 100 ml Sulfolan vorgelegt und 98,1 g (1,0 Mol) 100%ige $H_2SO_4$ zugetropft. Man heizt auf 180°C und destilliert in 70 Minuten das Reaktionswasser ab. Die Suspension wird abgesaugt und getrocknet. Ausbeute an p-Sulfanilsäure 96,2%.

Beispiel 28

94,0 g (1,01 Mol) Anilin werden in einer Mischung von 350 ml Tetralin und 150 ml Sulfolan vorgelegt und mit 98,1 g (1,0 Mol) 100%iger $H_2SO_4$ versetzt. Man heizt auf 180—200°C und destilliert in 50 Minuten das Reaktionswasser ab. Die Ausbeute an p-Sulfanilsäure beträgt 94,5%.

**Patentansprüche**

1. Verfahren zur Herstellung von Amino-arylsulfonsäuren mit freier oder neutralisierter Sulfonsäuregruppe, dadurch gekennzeichnet, daß in einem Reaktionsmedium, das Tetramethylensulfon und gegebenenfalls weitere inerte Lösungsmittel enthält, ein Arylamin der Formel

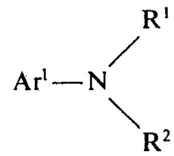

in der

$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und

$Ar^1$ das gegebenenfalls substituierte Benzol-, Naphthalin-, Anthracen-, Naphthochinon- oder Anthrachinongerüst oder das Gerüst eines aromatischen Heterocyclus darstellt,

mit einem Sulfonierungsagens bei einer Temperatur von 120 bis 280°C ohne Einsatz eines Katalysators umgesetzt wird.

2. Verfahren nach Anspruch 1, dadurch gekennzeichnet, daß das Reaktionsmedium 10 bis 2000 ml Tetramethylensulfon pro Mol Arylamin enthält.

3. Verfahren nach Anspruch 1 und 2, dadurch gekennzeichnet, daß das Reaktionsmedium 1 bis 99 Vol.-% weitere inerte Lösungsmittel neben dem Tetramethylensulfon enthält.

4. Verfahren nach Anspruch 1 und 2, dadurch gekennzeichnet, daß in Tetramethylensulfon als Reaktionsmedium gearbeitet wird.

5. Verfahren nach Anspruch 1 bis 4, dadurch gekennzeichnet, daß 0,5 bis 2,0 Mol Sulfonierungsagens pro Mol Arylamin eingesetzt werden.

6. Verfahren nach Anspruch 1 bis 5, dadurch gekennzeichnet, daß 0,95 bis 1,05 Mol Sulfonierungsagens pro Mol Arylamin eingesetzt werden.

7. Verfahren nach Anspruch 1 bis 6, dadurch gekennzeichnet, daß ein Arylamin der Formel

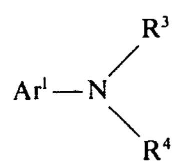

eingesetzt wird, worin

Ar[1] die in Anspruch 1 genannte Bedeutung hat und
$R^3$ und $R^4$ unabhängig voneinander Wasserstoff oder Alkyl bedeuten.

8. Verfahren nach Anspruch 1 bis 6, dadurch gekennzeichnet, daß ein Arylamin der Formel

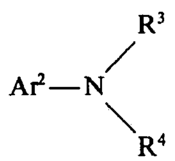

eingesetzt wird, worin

$Ar^2$ das gegebenenfalls substituierte Benzol- oder Naphthalingerüst bedeutet und
$R^3$ und $R^4$ unabhängig voneinander für Wasserstoff oder Alkyl stehen.

9. Verfahren nach Anspruch 1 bis 8, dadurch gekennzeichnet, daß als Sulfonierungsagens mindestens eines aus der Gruppe Schwefelsäure, $SO_3$ und Chlorsulfonsäure eingesetzt wird, wobei beim Einsatz von Schwefelsäure allein diese einen Wassergehalt von 0 bis 30 Gew.-% haben kann.

10. Verfahren nach Anspruch 1 bis 9, dadurch gekennzeichnet, daß das Reaktionsmedium, das nach beendeter Reaktion durch Abtrennung von der gebildeten Amino-arylsulfonsäure in freier oder salzartiger Form zurückgewonnen wird, ohne weitere Reinigung mindestens teilweise in die Reaktion zurückgeführt wird.

**Claims**

1. Process for the preparation of aminoarylsulphonic acids having a free or neutralised sulphonic acid group, characterised in that an arylamine of the formula

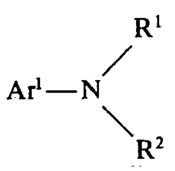

in which

$R^1$ and $R^2$ independently of one another denote hydrogen, alkyl, aralkyl or aryl, or both together form, together with the N atom on which they are substituents, a nitrogen-containing heterocyclic structure and
$Ar^1$ represents an optionally substituted benzene, naphthalene, anthracene, naphthoquinone or anthraquinone skeleton or the skeleton of an aromatic heterocyclic structure,

is reacted at a temperature of 120 to 280° C with a sulphonating agent in a reaction medium which contains tetramethylene sulphone and, if appropriate, other inert solvents, without the use of a catalyst.

2. Process according to Claim 1, characterised in that the reaction medium contains 10 to 2,000 ml of tetramethylene sulphone per mol of arylamine.

3. Process according to Claims 1 and 2, characterised in that the reaction medium contains in addition to tetramethylene sulphone 1 to 99% by volume of other inert solvents.

4. Process according to Claims 1 and 2, characterised in that tetramethylene sulphone is used as the reaction medium.

5. Process according to Claims 1 to 4, characterised in that 0.5 to 2.0 mols of sulphonating agent are used per mol of arylamine.

6. Process according to Claims 1 to 5, characterised in that 0.95 to 1.05 mols of sulphonating agent are used per mol of arylamine.

7. Process according to Claims 1 to 6, characterised in that an arylamine of the formula

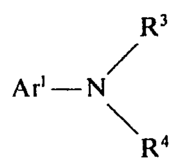

is used, wherein

$Ar^1$ has the meaning mentioned in Claim 1 and
$R^3$ and $R^4$ independently of one another denote hydrogen or alkyl.

8. Process according to Claims 1 to 6, characterised in that an arylamine of the formula

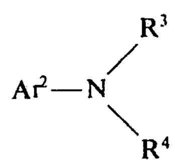

is used, wherein

$Ar^2$ denotes an optionally substituted benzene or naphthalene skeleton and
$R^3$ and $R^4$ independently of one another represent hydrogen or alkyl.

9. Process according to Claims 1 to 8, characterised in that the sulphonating agent used is at least one member of the group comprising sulphuric acid, $SO_3$ and chlorosulphonic acid, it being possible when using sulphuric acid alone for it to have a water content of 0 to 30% by weight.

10. Process according to Claims 1 to 9, characterised in that at least some of the reaction medium, which is recovered, after the reaction is complete, by separation from the aminoarylsul-

phonic acid formed, which is in a free or salt-type form, is passed back into the reaction without further purification.

**Revendications**

1. Procédé de production d'acides aminoarylsulfoniques à groupe acide sulfonique libre ou neutralisé, caractérisé en ce qu'on fait réagir avec un agent de sulfonation à une température de 120 à 280°C sans utiliser de catalyseur, dans un milieu de réaction qui contient de la tétraméthylènesulfone et, le cas échéant, d'autres solvants inertes, une arylamine de formule

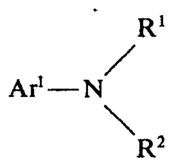

dans laquelle

$R^1$ et $R^2$ désignent, indépendamment l'un de l'autre, de l'hydrogènne, un groupe alkyle, aralkyle ou aryle, ou forment tous deux, conjointement avec l'atome d'azote qu'ils substituent, un hétérocycle azoté et

$Ar^1$ représente le squelette éventuellement substitué du benzène, du naphtalène, de l'anthracène, de la naphtoquinone ou de l'anthraquinone ou le sqelette d'un hétérocycle aromatique,

2. Procédé selon la revendication 1, caractérisé en ce que le milieu de réaction contient 10 à 2000 ml de tétraméthylènesulfone par mole d'arylamine.

3. Procédé suivant les revendications 1 et 2, caractérisé en ce que le milieu de réaction contient 1 à 99% en volume d'autres solvants inertes, à côté de la tétraméthylànesulfone.

4. Procédé suivant les revendications 1 et 2, caractérisé en ce qu'on opère dans la tétraméthylènesulfone comme milieu réactionnel.

5. Procédé suivant les revendications 1 à 4, caractérisé en ce qu'on utilise 0,5 à 2,0 moles d'agent de sulfonation par mole d'arylamine.

6. Procédé suivant les revendications 1 à 5, caractérisé en ce qu'on utilise 0,95 à 1,05 mole d'agent de sulfonation par mole d'arylamine.

7. Procédé suivant les revendications 1 à 6, caractérisé en ce qu'on utilise une arylamine de formule

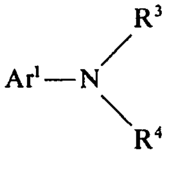

dans laquelle

$Ar^1$ a la définition mentionnée dans la revendication 1 et

$R^3$ et $R^4$ représentent indépendamment l'un de l'autre l'hydrogène ou un groupe alkyle.

8. Procédé suivant les revendications 1 à 6, caractérisé en ce qu'on utilise une arylamine de formule

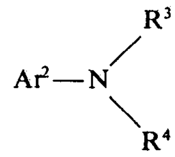

dans laquelle

$Ar^2$ désigne le squelette de benzène ou de naphtalène éventuellement substitué et

$R^3$ et $R^4$ représentent indépendamment l'un de l'autre l'hydrogène ou un groupe alkyle.

9. Procédé suivant les revendications 1 à 8, caractérisé en ce qu'on utilise comme agent de sulfonation au moins un composé choisi dans le groupe comprenant l'acide sulfurique, $SO_3$ et l'acide chlorosulfonique, et dans le cas de l'utilisation de l'acide sulfurique seul, cet acide peut avoir une teneur en eau de 0 à 30% en poids.

10. Procédé suivant les revendications 1 à 9, caractérisé en ce que le milieu de réaction qui est récupéré une fois la réaction terminée, par séparation de l'acide amino-arylsulfonique formé sour la forme libre ou sous la forme d'un sel, est recyclé au moins en partie dans la réaction sans autre purification.